# EUROPÄISCHE PATENTSCHRIFT

(11) **EP 0 537 608 B1**
(45) Veröffentlichungstag und Bekanntmachung des Hinweises auf die Patenterteilung: **21.02.1996**
(21) Anmeldenummer: 92117108.8
(22) Anmeldetag: 07.10.1992
(51) Int. Cl.: C07C 219/10

(54) **Verfahren zur technischen Herstellung von 2,2-Diphenyl-2-(2-ethyl-butoxy)-essigsäure-(2-dimethylamino-ethyl)-ester-hydrochlorid**
Process for the technical preparation of 2,2-diphenyl-2-(2-ethyl-butoxy)acetic acid, 2-dimethyl-amino-ethyl ester, hydrochloride
Procédé pour la préparation technique de la 2-diméthylamino-éthyl ester de l'acide 2,2-diphényl-2-(2-éthyl-butoxy)-acétique, chlorhydrate

(30) Priorität: 11.10.1991 DE 4133785; 17.07.1992 DE 4223688
(43) Veröffentlichungstag der Anmeldung: 21.04.1993
(73) Patentinhaber: APOGEPHA ARZNEIMITTEL GmbH, D-01309 Dresden (DE)
(72) Erfinder: Heschel, Michael, Dr., D-01796 Pirna (DE); Scheithauer, Steffen, Dr., D-01324 Dresden (DE); Otto, Rosemarie, D-01309 Dresden (DE); Starke, Christian, Dr., D-01309 Dresden (DE)
(74) Vertreter: Uhlemann, Henry, Dipl. Chem.

(56) Entgegenhaltungen:
- EP-A- 0 180 416
- DD-A- 106 643
- DE-B- 1 036 270
- US-A- 2 394 770
- CHEMICAL ABSTRACTS, vol. 102, no. 5, 4. Februar 1985, Columbus, Ohio, US; abstract no. 45638e, H. GOLDNER ET. AL. 'alpha,alpha-Diphenyl alpha-(2-ethylbutoxy)acetic acid beta-dimethylaminoethyl ester hydrochloride' Seite 545 ;Spalte 1 ;

## Beschreibung

Die Erfindung betrifft ein verbessertes Verfahren zur Herstellung von 2,2-Diphenyl-2-(2-ethyl-butoxy)-essigsäure-(2-dimethylamino-ethyl)-ester-hydrochlorid (DENAVERIN-Hydrochlorid) in arzneibuchgerechter Qualität und der dafür notwendigen Zwischenverbindungen unter technisch und ökologisch relevanten Bedingungen.

DENAVERIN-Hydrochlorid findet als Wirkstoff eines Human- und Veterinärpharmakons Verwendung.

Bekannt sind verschiedene Methoden zur Herstellung von DENAVERIN-Hydrochlorid, die aber aus technologischen und ökologischen Gründen technisch ungeeignet sind. Im weiteren ist die nach diesen Methoden erhaltene, für eine pharmazeutische Verwendung nur unzureichende Reinheit aufweisende Qualität des Endproduktes nachteilig.

Ausgehend von der bekannnten Tatsache, daß bei Carbonsäuren mit einer an ein quartäres C-Atom gebundenen Carboxylgruppe Veresterungen und Umesterungen im Gegensatz zu Alkylierungen erschwert sind, wurde die in der DD-PS 106 643 beschriebene Umesterungsmethode für DENAVERIN-Hydrochlorid in der DD-PS 211 336 als arelevant verworfen. Zu einem früheren Zeitpunkt wurde bereits darauf hingewiesen, daß die mit ca. 70 bis 80%iger Ausbeute verlaufende Alkylierung der Benzilsäure generell vorteilhafter als die 55 % an Ausbeute erzielende Umesterung mit Aminoalkoholen ist (J. prakt. Chem. 16 (1962) 258). Auf diesen Erkenntnissen aufbauend wurde der derzeitig bekannte dreistufige Reaktionsverlauf, die Alkylierung von Benzilsäure mit 2-Dimethylamino-ethylchlorid zum 2,2-Diphenyl-2-hydroxy-essigsäure-(2-dimethylamino-ethyl)-ester, dessen Chlorierung mittels Thionylchlorid zum 2,2-Diphenyl-2-chlor-essigsäure-(2-dimethylamino-ethyl)-ester-hydrochlorid und schließlich die Substitution des α-Chloratoms mittels 2-Ethyl-butanol zu 2,2-Diphenyl-2-(2-ethyl-butoxy)-essigsäure-(2-dimethylamino-ethyl)-ester-hydrochlorid (DENAVERIN-Hydrochlorid), entwickelt.

Für die Alkylierung von Benzilsäure oder Kaliumbenzilat mittels 2-Dimethylamino-ethylchlorid oder analogen Dialkylaminoalkylchloriden sind mehrere Verfahren bekannt, beispielsweise die Umsetzung in Isopropanol (J. prakt. Chem. 16 (1962) 258, 21 (1963) 1, 26 (1964) 32, 35 (1967) 313, 37 (1968) 143; Archiv Pharm. 288 (1955) 42, 75; J. Am. Chem. Soc. 65 (1943) 1967; DD-PS 24 310; DD-PS 26 909; DD-PS 28 209; DE-PS 1 194 870; DE-PS 1 251 333), in Aceton oder Ethylacetat (Helv. Chim. Acta 30 (1947) 295), in Chlorbenzol (Zh. Obsch. Khim 25 (1955) 2132) oder in Toluol (DD-PS 24 282; J. prakt. Chem. 16 (1962) 258; J. Chem. Soc. 1950 2887).

Lange Reaktionszeiten in Isopropanol und Aceton bzw. Ethylacetat bis zu 24 Stunden, durch hohe Reaktionstemperaturen bedingte Verfärbung bei der Verwendung des ökologisch bedenklichen Chlorbenzols und durch ohne einen Basenzusatz erhaltene Gemische von Benzilsäureesterhydrochlorid und Dialkylaminoalkylchlorid-hydrochloriden sind charakteristisch. Bei der Zugabe anorganischer Basen oder ausgehend von Kaliumbenzilat (vgl. J. Am. Chem. Soc. 65 (1943) 1967) erhält man den Benzilsäureester als freie Base. Als optimal wurde bisher das System Toluol o. Xylol/Kalium- o. Calciumcarbonat angesehen (DD-PS 24 782); J. prakt. Chem. 16 (1962) 258), dessen Nacharbeitung aber nichtreproduzierbare Ergebnisse liefert. Die durch eine oberflächliche Verkrustung des Carbonats mit dem entsprechenden Chlorid bedingte starke Verklumpung verhindert eine eindeutige und vollständige Reaktion. Das entstehende Reaktionswasser bewirkt eine sichtbare Azeotropbildung, welche destillativ durch die Flüchtigkeit des Chloralkylamins nicht zu beseitigen ist, den Reaktionsablauf durch Hydrolyseerscheinungen nachhaltig behindert und den resultierenden Benzilsäureester nur als gelb bis braun gefärbtes Öl liefert, der nur über sein Hydrochlorid isolierbar ist.

Allen bisherigen Verfahren gemeinsam ist die Verwendung der freien, durch Extraktionsoperationen erhaltenen Base des Dialkylaminoalkylchlorids. Die Herstellung von 2-Dimethylamino-ethylchlorid aus seinem Hydrochlorid ist der technologische Schlüsselschritt der bisherigen ersten Reaktionsstufe. Im Gegensatz zu 2-Dimethylamino-ethylchlorid-hydrochlorid ist aber dessen freie Base eine nur schlecht zu handhabende Chemikalie. So wird diese freie Base in Wasser, wäßriger Lauge oder wäßrigem Aceton bereits bei 60 °C innerhalb 30 Minuten vollständig hydrolysiert, dimerisiert innerhalb 2 Stunden bei Raumtemperatur, bildet mit Ether innerhalb kurzer Zeit unlösliche Nebenprodukte, destilliert durch ihre große Flüchtigkeit bereits mit Ether als Azeotrop über und liefert somit nur ca. 49 % an freier Base (vgl. J. Am. Chm. Soc. 73 (1953) 24). Extraktionsverfahren mittels Toluol sind zwar günstiger, aber ebenfalls mit einer Reihe von Nachteilen behaftet. Emulsionserscheinungen, sowohl in der wäßrigen als auch toluolischen Phase erschweren eine technisch einfache Phasentrennung. Wird auf die technisch aufwendige Trocknung der Toluolphase verzichtet, setzen sich nach längerem Stehen Restwassermengen ab, die infolge Hydrolyse zu Trübungen führen. Im weiteren ist der nicht zu vermeidende Resttoluolgehalt der wäßrigen Phase ökologisch nachteilig.

Für eine technisch einfache Verfahrensweise sollte somit eine extraktive Freisetzung des 2-Dimethylamino-ethylchlorid-hydrochlorids und der zusätzliche Einsatz von anorganischen Basen vermieden werden. Unter Umgehung dieser Nachteile wurde in einem Laborverfahren Kaliumbenzilat bzw. Benzilsäure/Kaliumcarbonat und 2-Dimethylamino-ethylchlorid-hydrochlorid bei 130 bis 140 °C zusammengeschmolzen und 8 bis 12 Stunden mit Isopropanol extrahiert (J. Am. Chem. Soc. 64 (1942) 428; Helv. Chim. Acta 34 (1951) 373). Dieses Verfahren liefert zwar das Hydrochlorid des Benzilsäureesters, ergibt aber bereits unter Laborbedingungen nicht reproduzierbare Ausbeuten und hat keine technische Relevanz.

Für die im zweiten Reaktionsschritt erfolgende Chlorierung zum 2,2-Diphenyl-2-chloressigsäure-(2-dimethylamino-ethyl)-ester-hydrochlorid mittels Thionylchlorid wird in allen bisher bekannten Verfahren die thermisch instabile, freie Base des Benzilsäureesters in inerten Lösungsmitteln, beispielsweise in Benzol, Chlorbenzol, o-Dichlorbenzol oder auch halogenierten, aliphatischen Kohlenwasserstoffen (vgl. DD-PS 211 336) eingesetzt. Neben diesen ökologisch zu vermeidenden Lösungsmitteln ist diese Verfahrensweise mit einer Reihe weiterer Nachteile behaftet. So entsteht bei der Chlorierung der freien Base innerhalb kurzer Zeit generell ein unbekannter gelber Fleck im Dünnschichtchromatogramm, der durch mehrmaliges Umkristallisieren des Endproduktes nicht zu beseitigen ist. Eine teilweise Entfernung dessen ist lediglich durch Reinigung des Endproduktes über die freie Base möglich. Diese extraktive Reinigung im System Alkalilauge/Chloroform erfordert einen hohen technologischen Aufwand durch Extraktion, Trocknung der organischen Phase, erneuter Hydrochloridbildung und erneuter Umkristallisation und führt zu einer nicht vertretbaren Ausbeuteverminderung an DENAVERIN-Hydrochlorid. Da der Benzilsäureester nur unter großem Ausbeuteverlust umzukristallisieren ist, aus Gründen des Arbeitsschutzes und möglicher Nebenreaktionen das α-Chlorbenzilsäureesterhydrochlorid nicht umkristallisiert werden sollte, wird bei allen bisher bekannten Verfahren ohne jegliche Zwischenreinigung gearbeitet. Nacharbeitungen ergaben, daß neben den in der DD-PS 211 336 aufgeführten Restmenge von Benzilsäureester, α-Chlorbenzilsäureesterhydrochlorid und dem bekannten gelben Fleck weitere zwei bis drei unbekannte Flecke im Dünnschichtchromatogramm des Endproduktes auftreten.

Für die am α-Chlorbenzilsäureesterhydrochlorid anzuschließende Substitution des α-Chloratomes mittels 2-Ethyl-butanol sind mehrere Verfahren bekannt, beispielsweise die Umsetzung in 2-Ethyl-butanol bei 90 bis 150 °C unter Herausblasen entstehenden Chlorwasserstoffs (DD-PS 31 921; DE-PS 1 232 962), unter Vakuum bei 130 °C (DD-PS 106 643) und gegebenenfalls durch Zusatz anorganischer Basen (Archiv Pharm. 288 (1955) 42, 75; DD-PS 26 909 (1964)). Die Nacharbeitung dieser Verfahren befriedigt nicht. Insbesondere ist der Einsatz anorganischer Basen durch keinerlei Verkürzung der Reaktionszeiten, Bildung von ständig schäumenden, azeotropen Gemischen, die zusätzlich zu Nebenprodukten führen und durch eine unvollständige Reaktion gekennzeichnet sind, nicht zu empfehlen. Als bisher optimal kann die in der DD-PS 211 336 beschriebene Verwendung von Co-Solventien angesehen werden. Die hierin verwendeten, mit Wasser nicht mischbaren, über 130 °C siedenden Lösungsmittel Chlorbenzol oder Xylol führen aber bei Reaktionstemperaturen von 140 bis 145 °C zu Gelbfärbungen, die in das Endprodukt übergehen. Die anschließende Verwendung von Butylacetat oder Butylacetatmischungen als letztes Lösungsmittel unter notwendigem Zusatz von Aktivkohle ist für ein geruchsfreies, leicht zu trocknendes und nur einen minimalen Ascheanteil enthaltendes Endprodukt unzweckmäßig.

Bedingt durch erhebliche Mängel bisheriger Verfahren zur Herstellung von DENAVERIN-Hydrochlorid besteht aufgrund der pharmazeutischen Wirksamkeit dieser Verbindung ein Bedürfnis an einem technologisch und ökologisch effizienten Herstellungsverfahren.

Aufgabe der Erfindung ist es, arzneibuchgerechtes DENAVERIN-Hydrochlorid in technisch einfacher Art und Weise, mit technologisch geringem Aufwand unter ökologisch akzeptablen Bedingungen in hoher Ausbeute und Reinheit herzustellen. Erfindungsgemäß wird die Aufgabe dadurch gelöst, daß Benzilsäure oder Kaliumbenzilat mit 2-Dimethylamino-ethylchlorid-hydrochlorid unter Zusatz tertiärer, aliphatischer oder cycloaliphatischer Amine mit einem pKₛ-Wert > 9 unter Gewinnung des Hydrochlorids der tertiären Amine zum 2,2-Diphenyl-2-hydroxy-essigsäure-(2-dimethylamino-ethyl)-ester (I) umgesetzt, dieser Ester (I) in Gegenwart primärer, sekundärer oder tertiärer Alkohole mittels Chlorwasserstoff oder konzentrierter Salzsäure in 2,2-Diphenyl-2-hydroxy-essigsäure-(2-dimethylamino-ethyl)-ester-hydrochlorid (II) überführt, dieses Hydrochlorid (II) mittels Thionylchlorid quantitativ, in 2,2-Diphenyl-2-chlor-essigsäure-(2-dimethylamino-ethyl)-ester-hydrochlorid (III) überführt und im letzten Reaktionsschritt dieses Hydrochlorid (III) mit 2-Ethyl-butanol in Gegenwart eines mit Wasser nicht mischbaren, unter 130 °C siedenden, inerten Lösungsmittels bei Reaktionstemperaturen von 120 bis 132 °C zum 2,2-Diphenyl-2-(2-ethyl-butoxy)-essigsäure-(2-dimethylamino-ethyl)-ester-hydrochlorid (IV) umgesetzt wird.

Im Gegensatz zu bisherigen Verfahren wird erfindungsgemäß im ersten Reaktionsschritt 2-Dimethylamino-ethylchlorid-hydrochlorid ohne zusätzliche Extraktionsoperationen direkt zur Alkylierung von Benzilsäure oder Kaliumbenzilat eingesetzt. Dabei ist es möglich, das im System Toluol/Thionylchlorid in quantitativer Ausbeute und genügender Reinheit anfallende Aminhydrochlorid auch als toluolfeuchtes Rohprodukt einzusetzen.

Überraschenderweise wurde gefunden, daß tertiäre, aliphatische oder cycloaliphatische Amine mit einem pkₛ-Wert > 9, beispielweise Triethylamin, Trimethylamin, N-Methylpyrrolidin, N-Methylpiperidin, N-Ethylpiperidin u. a. in aprotischen, apolaren, nicht aber in protischen, polaren und aprotischen, polaren Lösungsmitteln, vorzugsweise in Toluol oder Xylol, eine größere Basizität als 2-Dimethylamino-ethylchlorid und 2,2-Diphenyl-2-hydroxy-essigsäure-(2-dimethylamino-ethyl)-ester (I) zeigen. Somit ermöglichen sie eine quantitative Freisetzung des Aminhydrochlorids und eine eindeutige Bildung der freien Base des Benzilsäureesters (I) ohne die bekannten Nachteile heterogener, anorganischer Basen. Das erfindungsgemäße Arbeiten in aprotischen, apolaren Lösungsmitteln oder in den tertiären Aminen selbst unter wasserfreien Bedingungen bei Temperaturen von 50 bis 150 °C, im allgemeinen bei entsprechender Rückflußtemperatur, verhindert hydrolytisch bedingte Nebenreaktionen des Aminhydrochlorids und führt zu einer hohen Ausbeute an Benzilsäureester (I). Da das während der Reaktion heterogen ausfallende Hydrochlorid des eingesetzten tertiären Amins ständig aus dem Gleichgewicht entfernt wird, sind verkürzte Reaktionszeiten möglich. Unter dünnschichtchromatographischer Kontrolle ist ersichtlich, daß die Reaktion im wesentlichen nach zwei Stunden beendet ist, aus Gründen des suspendierten Chloralkylaminhydrochlorids aber 4 bis 6 Stunden, vorzugsweise 5 Stunden betragen sollte. Die Menge an 2-Dimethylamino-ethylchlorid-hydrochlorid, bezogen auf die eingesetzte Menge an Benzilsäure oder Kaliumbenzilat, beträgt vorteilhafterweise 1,1 bis 1,5, vorzugsweise 1,3 Äquivalente. Ein Überschuß von 1 bis 20 %, vorzugsweise 5 bis 10 % an tertiärem Amin, bezogen auf die Menge an eingesetztem Aminhydrochlorid und eingesetzter Benzilsäure bzw. nur bezogen auf die Menge an eingesetztem Aminhydrochlorid im Falle der Verwendung von Kaliumbenzilat, erweist sich als optimal. Das in der Reaktion anfallende, vollkommen reine, tertiäre Aminhydrochlorid bzw. das Gemisch mit Kaliumchlorid kann nach Trocknung in üblicher Weise mit konzentrierten Laugen problemlos zu wiederverwendbarem Amin aufgearbeitet werden. Überraschenderweise wurde im weiteren gefunden, daß 2-Dimethylamino-ethylchlorid unter wasserfreien Bedingungen in aprotischen, apolaren Lösungsmitteln über längere Zeit, beispielsweise über 2 Monate bei Raumtemperatur, stabil bleibt. Somit lassen sich die nach Einengen auf den Benzilsäureester-(I)-Rückstand anfallenden, aminhaltigen Lösungsmittel nach gaschromatographischer Bestimmung der Restamingehalte ohne weitere Aufarbeitung unter Erhalt beider, im Überschuß vorliegender Amine erneut einsetzen und ermöglichen einen technologisch vorteilhaften Lösungsmittelkreislauf. Das nach dieser Art und Weise erhaltene Rohprodukt an Benzilsäureester (I) enthält nach dünnschichtchromatographischer Auswertung bis auf die 0,1 bis 3 % nicht umgesetzte Benzilsäure und gegebenenfalls bereits in der eingesetzten Benzilsäure vorhandenen Benzophenonreste(1 bis 2%) keine weiteren Verunreinigungen.

Erfindungsgemäß wird die Aufgabe im weiteren dadurch gelöst, daß im Gegensatz zu bisher bekannten Verfahren der Benzilsäureester (I) durch Lösen in primären, sekundären oder auch tertiären Alkoholen mittels Chlorwasserstoff, der entweder in situ erzeugt oder direkt eingeleitet wird, oder auch mittels konzentrierter Salzsäure in hoher Ausbeute unter Einstellung eines pH-Wertes auf 1 bis 3 in sein Hydrochlorid, das 2,2-Diphenyl-2-hydroxy-essigsäure-(2-dimethylamino-ethyl)-ester-hydrochlorid (II) überführt wird. Im Falle der Verwendung von gasförmigem Chlorwasserstoff wird vorzugsweise Isopropanol, im Falle der Verwendung von konzentrierter Salzsäure dagegen Butanol eingesetzt. Um Ausbeuteverminderungen und thermisch bedingte, säurekatalysierte Verseifungen des Benzilsäureesters (I) zu vermeiden, wird im letzten Fall günstiger im Vakuum bei Temperaturen zwischen 50 bis 70 °C unter azeotroper Abdestillation des Wasseranteils gearbeitet.

Im weiteren wurde gefunden, daß das in inerten, aromatischen Verdünnungsmitteln, beispielsweise Toluol oder Xylol, ständig heterogen suspendierte Benzilsäureesterhydrochlorid (II) im nächsten Reaktionsschritt, der Chlorierung mittels Thionylchlorid zum 2,2-Diphenyl-2-chlor-essigsäure-(2-dimethylamino-ethyl)-ester-hydrochlorid (III) bei Temperaturen zwischen 70 bis 100 °C, vorzugsweise bei 90 bis 95 °C, innerhalb kurzer Zeit eine quantitative Reaktion ergibt. Für eine hohe Ausbeute und Reinheit des α-Chlorbenzilsäureesterhydrochlorids (III) wird Thionylchlorid in Mengen von 1,5 bis 2,0 , vorzugsweise in Mengen von 1,7 Äquivalenten, bezogen auf eingesetztes Benzilsäureesterhydrochlorid (II), verwendet. Das durch den erfindungsgemäßen Einsatz von Benzilsäureesterhydrochlorid (II) anstatt der freien Base des Benzilsäureesters (I) fast quantitativ anfallende α-Chlorbenzilsäureesterhydrochlorid (III) enthält im Gegensatz zu bisher bekannten Verfahren neben Restmengen von 0,3 bis 2 % an nicht umgesetzten Benzilsäureesterhydrochlorids (II) keine weiteren Verunreinigungen. Im weiteren ist durch die Verwendung von inerten, aromatischen Verdünnungsmitteln das ausfallende α-Chlorbenzilsäureesterhydrochlorid (III) ohne destillative Aufarbeitung durch technologisch einfache Trennverfahren isolierbar und kann lösungsmittelfeucht direkt im nächsten Reaktionsschritt eingesetzt werden. Das auf diese Art und Weise erhaltende α-Chlorbenzilsäureesterhydrochlorid (III) liefert im nächsten Reaktionsschritt ein qualitätsgerechtes Endprodukt unter Verzicht auf mehrmaliges, stark ausbeuteverminderndes Umkristallisieren oder die extraktive Reinigung über die freie Base. Das resultierende, thionylchloridhaltige Verdünnungsmittel ist technologisch vorteilhaft unter Erhalt des eingesetzten Thionylchloridüberschusses ohne destillative Aufarbeitung für 2 bis 3 weitere Ansätze einsetzbar. Erst danach empfiehlt sich eine einfache Normaldruckdestillation, um die generell bei der Verwendung von Thionylchlorid auftretenden gelblichen Verfärbungen zu beseitigen.

Neben der Chlorierung in Co-Solventien erweist sich die Chlorierung in reinem Thionylchlorid als technologisch ebenfalls sehr günstig und führt zu einem reinweißen, kristallinen α-Chlorbenzilsäureesterhydrochlorid (III) mit einem Benzilsäureesterhydrochloridgehalt von 0,3-0,5 %. Hierzu wird die 1,9 - 2,1 fache Gewichtsmenge an Thionylchlorid, bezogen auf eingesetztes Benzilsäureesterhydrochlorid (II), vorgelegt und bei 0 °C bis 25 °C das Hydrochlorid (II) in fester Form zugegeben. Bereits bei Raumtemperatur erfolgt 98 %iger Umsatz zum α-Chlorbenzilsäureesterhydrochlorid (III). Für eine Umsetzungsrate von 99,5 bis 99,7 % ist ein zusätzliches Erwärmen unter Rückfluß notwendig. Nach der Reaktionszeit wird bei einer Heiztemperatur von maximal 110 bis 130 °C das Thionylchlorid destillativ entfernt, wodurch das α-Chlorbenzilsäureesterhydrochlorid (III) grobkristallin und reinweiß erhalten wird. Das abdestillierte Thionylchlorid zeigt keinerlei Verfärbungen und ist beliebig oft einsetzbar. Unter Ausnutzung der Apparatetemperatur kann sofort der nächste Reaktionsschritt angeschlossen werden.

Im letzten Reaktionsschritt, der Veretherung des α-Chlorbenzilsäureesterhydrochlorids (III) mittels 2-Ethyl-butanol zum 2,2-Diphenyl-2-(2-ethyl-butoxy)-essigsäure-(2-dimethylamino-ethyl)-ester-hydrochlorid (IV) werden erfindungsgemäß als Co-Solventien mit Wasser nichtmischbare, unter 130 °C siedende, inerte Lösungsmittel, vorzugsweise Toluol, eingesetzt, um durch verminderte Rückflußtemperaturen der Reaktionsgemische von 120 bis 132 °C Gelbfärbungen des Endproduktes zu vermeiden. Dabei wurde dünnschichtchromatographisch gefunden, daß nach einer Reaktionszeit von 8 Stunden keinerlei α-Chlorbenzilsäureesterhydrochlorid (III) mehr nachweisbar ist, daß der Anteil des im eingesetzten α-Chlorbenzilsäureesterhydrochlorid als Nebenprodukt enthaltenen Benzilsäureesterhydrochlorids (0,3 bis 2 %) während der gesamten Reaktionszeit konstant bleibt und daß unter diesen Reaktionsbedingungen keine Wasserbildung, möglich durch säurekatalysierte Wasserabspaltung von 2-Ethyl-butanol zu Butenderivaten, zu beobachten ist.

Schließlich wurde gefunden, daß es für ein geruchsfreies und technisch leicht zu trocknendes DENAVERIN-Hydrochlorid (IV) günstig ist, anstatt aus Butylacetat aus Methylethylketon, vorzugsweise der 1,6 bis 2,2-fachen Gewichtsmenge, unter Einstellung eines pH-Wertes von 2 bis 3, umzukristallisieren. Da das nach dem erfindungsgemäßen Verfahren hergestellte Endprodukt bereits eine hohe Reinheit aufweist, lassen sich durch einmalige Umkristallisation Nebenprodukte, beispielsweise die 0,3 bis 2 % an Benzilsäureesterhydrochlorid (II), vollständig entfernen und die Methylethylketon-Mutterlaugen ohne Zwischenreinigung mehrmals einsetzen.

Anhand nachfolgender Ausführungsbeispiele wird die Erfindung näher erläutert.

Die in den nachfolgend benannten Ausführungsbeispielen hergestellten und aufgeführten Verbindungen wurden dünnschichtchromatographisch bei folgenden Bedingungen untersucht:
- - DC-Fertigplatte:: Kieselgel 60 GF₂₅₄ (Merck)
- - Laufmittel(V %):: Chloroform (50); Cyclohexan (35); Methanol (15); NH₃ 6 molar (1,5)
- - Detektion:: 1. UV 2. 35%ige ethanolische Schwefelsäure; 30 Minuten bei 130 °C behandeln
- - Auftragungsmenge:: 200 ug

### Ausführungsbeispiel 1

### 2,2-Diphenyl-2-hydroxy-essigsäure-(2-dimethylamino-ethyl)-ester aus Benzilsäure

12,8 mol (1840 g) toluolfeuchtes Rohprodukt von 2-Dimethylamino-ethylchlorid-hydrochlorid (hergestellt in quantitativer Ausbeute in Toluol mit 1,5 mol Thionylchlorid bei 80 bis 90 °C) und 10 mol (2280 g) Benzilsäure werden in 10 bis 15 l absolutem oder andestilliertem Toluol vorgelegt und unter Rühren auf 60 °C erwärmt. Anschließend läßt man 23,94 mol (2422 g; 5 % Überschuß) Triethylamin zulaufen und rührt unter Rückfluß 5 Stunden. Es wird heiß von 3000 bis 3130 g (96 bis 100 %) ausgefallenem Triethylaminhydrochlorid abgesaugt, mit 1 bis 2 l möglichst warmem Toluol gewaschen und die Toluollösung im Vakuum auf Rückstand eingeengt. Entweder wird im gleichen Reaktionsgefäß sofort die Hydrochloridbildung angeschlossen oder man rührt zur Isolation der freien Base mit 3 bis 5 l Wasser durch, saugt scharf ab und läßt bei Raumtemperatur vollständig trocknen.
Ausbeute: 2550 bis 2780 g (85 bis 93 % d. Th.)
Fp = 87 bis 91 °C und 97 bis 101 °C
Dünnschichtchromatogramm: 2 % Benzilsäure (45,6 g) sind nicht umgesetzt; außer Benzophenon keine weiteren Verunreinigungen.

### Ausführungsbeispiel 2

### 2,2-Diphenyl-2-hydroxy-essigsäure-(2-dimethylamino-ethyl)-ester aus Kaliumbenzilat

Analog Beispiel 1 werden 10,1 mol (1455 g) toluolfeuchtes 2-Dimethylamino-ethylchlorid-hydrochlorid in 15 l Toluol, bestehend aus 12 l aminhaltiger Toluollösung des Beispiels 1 mit 2,7 mol an 2-Dimethylamino-ethylchlorid- und 0,7 mol an Triethylaminrestgehalt und 3 l frischem Toluol, mit 10 mol (2665 g) Kaliumbenzilat versetzt und auf 60 °C unter Rühren erwärmt. Anschließend läßt man 9,905 mol (1003 g; 5 % Überschuß) Triethylamin zulaufen und rührt 5 Std. unter Rückfluß nach. Es wird heiß von 2100 g (98,3 %) Triethylaminhydrochlorid/Kaliumchlorid-Gemisch abgesaugt und analog Beispiel 1 weiterverarbeitet.
Ausbeute: 2755 g (92 % d. Th.)
Fp= 86 bis 91 °C und 97 bis 100 °C
Dünnschichtchromatogramm: 0,2 % Benzilsäure; keine weiteren Verunreinigungen

### Ausführungsbeispiel 3

### 2,2-Diphenyl-2-hydroxy-essigsäure-(2-dimethylamino-ethyl)-ester-hydrochlorid mittels Chlorwasserstoff

Die 2550 bis 2780 g Benzilsäureester des Beispiels 1 oder 2 werden in 20 l Isopropanol bei 60 °C gelöst, wenn nötig filtriert, bzw. man versetzt im Reaktionsgefäß der Beispiele 1 oder 2 sofort mit diesen 20 l Isopropanol. Anschließend wird mit weiteren 15 l Isopropanol versetzt und unter Rühren auf 70 °C erwärmt. Ohne weiteres Heizen wird unter Rühren ein gerade absorbierbarer, kräftiger Chlorwasserstoffstrom eingeleitet, bis ein pH-Wert von 2 bis 3 konstant bleibt. Im Falle der in situ-Herstellung mit Thionylchlorid werden zuerst 84 ml Wasser und anschließend 525 g Thionylchlorid zugegeben. Durch die Reaktionsenthalpie kann leichter Rückfluß auftreten. Ein Teil des entstandenen Hydrochlorids kann bereits ausfallen, der auch unter Rückfluß nicht vollständig aufzulösen ist. Man läßt auf Raumtemperatur unter Intervallrühren abkühlen, saugt scharf ab, wäscht mit 1 bis 1,5 l Isopropanol und trocknet bei 110 °C. Das etwas saure Isopropanol wird ohne Reinigung erneut eingesetzt.
Ausbeute: 2650 bis 2910 g (78,9 bis 86,6 % d. Th. bezogen auf den Benzilsäure/Kaliumbenzilat-Einsatz)
Fp = 187 bis 191 °C
Dünnschichtchromatogramm: keine weiteren Verunreinigungen

### Ausführungsbeispiel 4

### 2,2-Diphenyl-2-hydroxy-essigsäure-(2-dimethylamino-ethyl)-ester-hydrochlorid mittels konzentrierter Salzsäure

1 mol (299 g) Benzilsäureester aus Beispiel 1 oder 2 werden in 600 bis 700 ml Butanol bei 60 °C gelöst, wenn nötig filtriert, mit 1,25 mol (124 g) 37 %iger Salzsäure bei ca. 50 °C versetzt und unter leichtem Vakuum bei ca. 60 °C am Wasserabscheider gerührt.

Wenn ca. 5 bis 10 ml Wasser abgeschieden sind, fällt das Hydrochlorid in Form von Nadeln aus. Nach 45 bis 60 Minuten sind ca. 85 % der theoretischen Wassermenge abgeschieden. Unter Normaldruck wird auf 90 °C erwärmt, wodurch sich das Hydrochlorid erneut auflöst. Anschließend wird unter Intervallrühren auf Raumtemperatur abgekühlt, abgesaugt und bei 110 °C getrocknet. Die resultierende Mutterlauge wird für einen weiteren Ansatz eingesetzt, danach destillativ aufgearbeitet und resultierende Restmengen an Hydrochlorid werden erneut umkristallisiert.
Ausbeute: 289 g (86 % d. Th. bezogen auf den eingesetzten Benzilsäureester)
Fp = 184 bis 187,5 °C
Dünnschichtchromatogramm: keine weiteren Verunreinigungen

### Ausführungsbeispiel 5

### 2,2-Diphenyl-2-chlor-essigsäure-(2-dimethylamino-ethyl)-ester-hydrochlorid

15 mol (5038 g) reines Benzilsäureesterhydrochlorid der Beispiele 3 oder 4 werden in 16 bis 16,5 l absolutem oder andestilliertem Toluol suspendiert, mit 10 ml DMF versetzt und auf eine Starttemperatur von 85 bis 95 °C gebracht. 25,5 mol (1,7 Äquivalente; 3034 g) Thionylchlorid werden ohne weiteres Heizen so unter die Oberfläche gepumpt, daß die Absorption beherrscht werden kann. Hierbei ist zu beachten, daß die Induktionsperiode 15 bis 30 Minuten betragen kann. Die weitere Thionylchloridzugabe ist relativ problemlos in 1 bis 2 Stunden möglich, da ein schnelles Wegreagieren des Thionylchlorids erfolgt. Zur Vervollständigung der Reaktion wird noch 3 bis 3,5 Std. bei 90 bis 95 °C nachgerührt. Nach Abkühlen auf Raumtemperatur wird im Falle einer Isolierung dieses Hydrochlorides scharf abgesaugt, mit 1 bis 2 l Isopropanol gewaschen, wobei eine separate Vorlage zu verwenden ist, und bei 110 °C getrocknet.
Günstiger ist es, daß α-Chlorbenzilsäureesterhydrochlorid toluolfeucht in der nächsten Reaktionsstufe einzusetzen. Hierzu wird entweder nur vom thionylchloridhaltigen Toluol abgesaugt oder empfehlenswert das Toluol mittels eines Saugrohres, welches mit einer G1-Fritte verschlossen ist, aus dem Reaktionsgefäß so weit wie möglich abgezogen. Gegebenenfalls können durch Anlegen von Vakuum und Aufheizen auf 40 bis 60 °C thionylchloridhaltige Toluolreste vollständig entfernt werden. Die nachfolgende Veretherung ist somit im gleichen Reaktionsgefäß ohne Isolierung des α-Chlorbenzilsäureesters möglich.
Ausbeute: 5208 g (98 % d. Th.)
Fp = 179 bis 181 °C
Dünnschichtchromatogramm: 2 % (100,8 g) Benzilsäureesterhydrochlorid sind nicht umgesetzt; keine weiteren Verunreinigungen.

Das nur leicht hellgelb gefärbte thionylchloridhaltige Toluol wird ohne Reinigung für einen weiteren Ansatz eingesetzt und auf 16 bis 16,5 l Volumen durch frisches Toluol ergänzt. 15 mol Benzilsäureesterhydrochlorid werden eingetragen, mit 10 ml DMF versetzt und unter Rühren langsam auf eine Starttemperatur von 75 bis 80 °C aufgeheizt. Die Induktionsperiode beträgt hier ebenfalls 15 bis 30 Minuten. Nachdem die Hauptmenge des noch vorhandenen Thionylchlorids wegreagiert ist (ca. 1 bis 1,5 Std.), werden analog voranstehend 15 mol (1785 g) Thionylchlorid innerhalb 1 bis 2 Stunden unter die Oberfläche gepumpt und im weiteren nach dem ersten Ansatz verfahren.
Ausbeute: 5155 g (97 % d. Th.)
Fp= 177 bis 183 °C
Dünnschichtchromatogramm: 1,5 % Benzilsäureesterhydrochlorid nicht umgesetzt; keine weiteren Verunreinigungen

### Ausführungsbeispiel 6

### 2,2-Diphenyl-2-chlor-essigsäure-(2-dimethylamino-ethyl)-ester-hydrochlorid

Ca. 9,58-10,58 kg bzw. 5,84-6,46 l Thionylchlorid werden vorgelegt, auf 0-25 °C abgekühlt und innerhalb 15 Minuten 15 mol (5038 g) Benzilsäureesterhydrochlorid in fester Form unter Rühren eingetragen. Nach dem exothermen Start wird beispielsweise innerhalb 45-60 Minuten auf 40-45 °C und in weiteren 60 Minuten auf die Rückflußtemperatur von 81-87 °C aufgeheizt. Zur Vervollständigung der Reaktion wird 3,5-4 Stunden unter Rückfluß gerührt. Nachdem 50-65 % des noch vorhandenen Thionylchlorids (ca. 2,4-3,5 l) bei Normaldruck abdestilliert sind, beginnt die Auskristallisation des α-Chlorbenzilsäureesterhydrochlorids in Form großer, weißer Kristalle. Dabei wird die Kristallisationsenthalpie durch Verminderung der Heizleistung abgefangen. Unter weiterem Rühren und Anlegen von Vakuum entsprechend der Destillationsgeschwindigkeit werden letzte Thionylchloridspuren beseitigt. Bei einer Badtemperatur von ca. 110-115 °C sollte ein Endvakuum von 10-15 Torr erreicht werden. Neben 0,3-0,5 % Benzilsäureesterhydrochlorid (II) sind im Dünnschichtchromatogramm keine weiteren Verunreinigungen enthalten. Unter Ausnutzung der Apparatetemperatur kann nach der quantitativen Umsetzung sofort der nächste Reaktionsschritt, die Veretherung angeschlossen werden.

### Ausführungsbeispiel 7

### 2,2-Diphenyl-2-(2-ethyl-butoxy)-essigsäure-(2-dimethylamino-ethyl)-ester-hydrochlorid

Die nach Ausführungsbeispiel 5 oder 6 erhaltenen 15 mol α-Chlorbenzilsäureesterhydrochlorid werden in einem Gemisch von 90 mol (9,2 kg bzw. 11,1 l) 2-Ethylbutanol und 45 mol (4,14 kg bzw. 4,8 l) Toluol 10-12 Stunden unter Rückfluß am Wasserabscheider gerührt, wobei sich ca. 45 ml verdünnte Salzsäure abscheiden. Nach erfolgter Reaktion wird bei einer Manteltemperatur von 120 bis 130 °C im Vakuum, am Ende beispielsweise bei 5-10 Torr, unter Rühren auf kristallinen Rückstand eingeengt, wodurch 96-98 % des restlichen Veretherungsgemisches als wiederverwendungsfähiges Destillat erhalten werden. Bezogen auf eingesetztes Benzilsäureesterhydrochlorid wird die 1,75fache Gewichtsmenge an Methylethylketon (8,82 kg bzw. 11 l) nach Verminderung der Manteltemperatur auf 90-95 °C zugegeben, ca. 15 Minuten bis zur vollständigen Auflösung unter Rückfluß gerührt, von mechanischen Verunreinigungen abfiltriert und mittels Chlorwasserstoff ein pH-Wert von 2-3 eingestellt. Unter Intervallrühren läßt man langsam auf Raumtemperatur abkühlen, wodurch bei ca. 35-55 °C die Auskristallisation beginnt. Durch Abzentrifugieren, Waschen mit ca. 1,5 l Methylethylketon und Trocknen bei 80-110 °C im Umluft- oder Vakuumtrockenschrank erhält man 4790-5230 g (76 bis 83 % d. Th.) an Denaverinhydrochlorid. Zur Ausbeuteerhöhung wird die leicht gelb gefärbte Methylethylketon-Mutterlauge für einen weiteren Ansatz eingesetzt, wodurch eine Ausbeute von 85-97 % d. Th. resultieren. Die nach der zweiten Verwendung resultierende Mutterlauge wird unter Normaldruck auf ein kristallisationsfähiges Restvolumen von 3-4 Litern eingeengt und liefert verunreinigtes Denaverinhydrochlorid, welches durch Zusatz von 10 g/l Aktivkohle in analoger Weise aus Methylethylketon umkristallisiert werden kann.

### Ausführungsbeispiel 8

### Umkristallisation von 2,2-Diphenyl-2-(2-ethyl-butoxy)-essigsäure-(2-dimethylamino-ethyl)-ester-hydrochlorid

Für eine pharmazeutische Qualität werden die Denaverinhydrochlorid-Rohprodukte gegebenenfalls in der 2,2fachen Gewichtsmenge Methylethylketon bei 60 °C unter Rühren suspendiert, durch Zugabe ethanolischer Salzsäure oder durch Einleiten von Chlorwasserstoff unter weiterem Aufheizen auf Rückflußtemperatur auf einen pH-Wert von 2 bis 3 eingestellt und gegebenenfalls filtriert. Man laßt einen Tag langsam auskristallisieren, zentrifugiert 1 bis 2 Stunden vom Lösungsmittel ab und wäscht mit Methylethylketon. Nach kurzem Antrocknen bei ca. 80 °C wird beispielsweise in einem FREWITT-Siebgranulierer zerkleinert, um später größere, steinharte Brocken zu vermeiden. Über Nacht wird im Vakuum- oder Umlufttrockenschrank bei ca. 80-110 °C getrocknet.
Ausbeute: 88 % bezogen auf eingesetztes Endprodukt
Fp= 142,5 bis 145,5 °C
Dünnschichtchromatogramm: keine weiteren Zusatzflecke

Die resultierende, leicht saure Methylethylketon-Mutterlauge (ca. 83-88 %) wird in analoger Weise erneut zur Umkristallisation eingesetzt.
Ausbeute nach zweimaliger Verwendung: 96 % bezogen auf eingesetztes Rohprodukt
Fp= 140 bis 143 °C
Dünnschichtchromatogramm: keine weiteren Zusatzflecke

## Patentansprüche

1. Verfahren zur technischen Herstellung von 2,2-Diphenyl-2-(2-ethyl-butoxy)-essigsäure-(2-dimethylamino-ethyl)-ester-hydrochlorid (IV) durch Alkylierung von Benzilsäure oder ihrer Alkali- oder Erdalkalisalze, Chlorierung des gebildeten Benzilsäureesters und Veretherung des α-Chlorbenzilsäureesters zum Endprodukt, dadurch gekennzeichnet, daß Benzilsäure oder deren Alkali- oder Erdalkalisalz mit 2-Dimethylamino-ethylchlorid-hydrochlorid unter Zusatz tertiärer, aliphatischer oder cycloaliphatischer Amine mit einem pKₛ-Wert > 9 unter Gewinnung des Hydrochlorids der tertiären Amine zum 2,2-Diphenyl-2-hydroxy-essigsäure-(2-dimethylamino-ethyl)-ester (I) umgesetzt, dieser Ester (I) in Gegenwart primärer, sekundärer oder tertiärer Alkohole mittels Chlorwasserstoff oder konzentrierter Salzsäure in 2,2-Diphenyl-2-hydroxyessigsäure-(2-dimethylamino-ethyl)-ester-hydrochlorid (II) überführt, dieses Hydrochlorid (II) mittels Thionylchlorid quantitativ in 2,2-Diphenyl-2-chlor-essigsäure-(2-dimethylamino-ethyl)-ester-hydrochlorid (III) überführt wird und im letzten Reaktionsschritt dieses Hydrochlorid (III) mit 2-Ethyl-butanol in Gegenwart eines mit Wasser nicht mischbaren, unter 130 °C siedenden, inerten Lösungsmittels bei Reaktions-temperaturen von 120 bis 132 °C zum 2,2-Diphenyl-2-(2-ethyl-butoxy)-essigsäure-(2-dimethylamino-ethyl)-ester-hydrochlorid (IV) umgesetzt wird.

2. Verfahren nach Anspruch 1, dadurch gekennzeichnet, daß 1,1 bis 1,5 Äquivalente an 2-Dimethylamino-ethylchlorid-hydrochlorid, bezogen auf die Menge an eingesetzter Benzilsäure bzw. deren Salz eingesetzt werden.

3. Verfahren nach Anspruch 1, dadurch gekennzeichnet, daß Benzilsäure oder deren Salz mit 2-Dimethylamino-ethylchlorid-hydrochlorid unter Zusatz tertiärer, aliphatischer oder cycloaliphatischer Amine mit einem pKₛ-Wert > 9 gegebenenfalls in Gegenwart aprotischer, apolarer Lösungsmittel zum 2,2-Diphenyl-2-hydroxy-essigsäure-(2-dimethylamino-ethyl)-ester (I) umgesetzt wird.

4. Verfahren nach Anspruch 1, dadurch gekennzeichnet, daß als tertiäres Amin Triethylamin mit einem Überschuß von 5 bis 10 %, bezogen auf die gesamte, zu bindende Chloridmenge, eingesetzt wird.

5. Verfahren nach Anspruch 1, dadurch gekennzeichnet, daß für den ersten Reaktionsschritt als Lösungsmittel Toluol oder Xylol eingesetzt und die Reaktion im Temperaturbereich von 50 bis 150 °C, im allgemeinen unter Rückfluß, durchgeführt wird.

6. Verfahren nach Anspruch 1, dadurch gekennzeichnet, daß bei der Überführung des Benzilsäureesters (I) in sein Hydrochlorid mittels Chlorwasserstoff, der wahlweise in situ erzeugt oder direkt eingeleitet bzw. zugegeben, in Gegenwart des Lösungsmittels Isopropanol und bei Verwendung von konzentrierter Salzsäure als Lösungsmittel Butanol verwendet und hierbei im leichten Vakuum bei Temperaturen von 50 bis 70 °C unter azeotroper Abdestillation des Wassergehaltes und unter Einstellung eines pH-Wertes von 1 bis 3 gearbeitet wird.

7. Verfahren nach Anspruch 1, dadurch gekennzeichnet, daß das 2,2-Diphenyl-2-hydroxyessigsäure-(2-dimethylamino-ethyl)-ester-hydrochlorid unter heterogenen Bedingungen in aromatischen, inerten Verdünnungsmitteln, vorzugsweise Toluol oder Xylol, mit 1,7 Äquivalenten an Thionylchlorid bei 90 bis 95 °C, quantitativ in das α-Chlorbenzilsäureesterhydrochlorid (III) überführt wird.

8. Verfahren nach Anspruch 1, dadurch gekennzeichnet, daß das 2,2-Diphenyl-2-hydroxyessigsäure-(2-dimethylamino-ethyl)-ester-hydrochlorid unter homogenen Bedingungen in der 1,9 bis 2,1fachen Gewichtsmenge an Thionylchlorid, bezogen auf die Menge an eingesetztem Benzilsäureesterhydrochlorid (II) bei 5 bis 90 °C quantitativ in das α-Chlorbenzilsäureesterhydrochlorid (III) umgesetzt wird.

9. Verfahren nach Anspruch 1, dadurch gekennzeichnet, daß für die Veretherung des α-Chlorbenzilsäureesterhydrochlorids (III) Toluol als Co-Solvenz eingesetzt wird.

10. Verfahren nach Anspruch 1, dadurch gekennzeichnet, daß das erhaltene Endprodukt (IV) aus der 1,6 bis 2,2-fachen Menge Methylethylketon unter Einstellung eines pH-Wertes von 2 bis 3 umkristallisiert wird.

## Claims

1. Process for the production of 2,2-diphenyl-2-(2-ethyl-butoxy)-acetic acid-(2-dimethylamino-ethyl)-ester-hydrochloride (IV) by alkalisation of benzilic acid or its alkali or alkaline earth salts, chlorination of the benzilic acid ester produced and etherisation of the α-chlorobenzilic acid ester to the final product, characterised in that benzilic acid or its alkali or alkaline earth salt is converted with 2-dimethylamino-ethyl-chloride-hydrochloride under the addition of tertiary, aliphatic or cyclo aliphatic amines with a pKₐ value > 9 under the production of hydrochloride of the tertiary amines into 2,2-diphenyl-2-hydroxy-acetic acid-(2-dimethylamino-ethyl)-ester (I), this ester (I) is converted in the presence of primary, secondary or tertiary alcohols using hydrogen chloride or concentrated hydrochloric acid into 2,2-diphenyl-2-hydroxy-acetic acid-(2-dimethylamino-ethyl)-ester-hydrochloride (II), this hydrochloride (II) is converted using thionyl chloride quantitatively into 2,2-diphenyl-2-chloro-acetic acid-(2-dimethylamino-ethyl)-ester-hydrochloride (III) and in the last stage of the reaction this hydrochloride (III) is converted with 2-ethyl-butanol in the presence of an inert solvent which is not soluble in water and boils at below 130°C into 2,2-diphenyl-2-(2-ethyl-butoxy)-acetic acid-(2-dimethylamino-ethyl)-ester-hydrochloride (IV) at reaction temperatures of 120 to 132°C.

2. Process according to claim 1, characterised in that 1.1 to 1.5 equivalents of 2-dimethylamino-ethyl-chloride-hydrochloride are used based on the quantity of benzilic acid or salts.

3. Process according to claim 1, characterised in that benzilic acid or its salts are converted with 2-dimethylamino-ethyl-chloride-hydrochloride under the addition of tertiary, aliphatic or cyclo aliphatic amines with a pKₐ value > 9 also in the presence of aprotic, apolar solvent into 2,2-diphenyl-2-hydroxy-acetic acid-(2-dimethylamino-ethyl)-ester (I).

4. Process according to claim 1, characterised in that triethylamine with an excess of 5 to 10% based on the total quantity of chloride to be bonded is used as the tertiary amine.

5. Process according to claim 1, characterised in that toluol or xylol is used as the solvent for the first reaction stage and the reaction is carried out in a temperature range of 50 to 150°C, generally with a return flow.

6. Process according to claim 1, characterised in that in the conversion of benzilic acid-ester (I) into its hydrochloride using the hydrogen chloride produced either in situ or added directly, in the presence of the solvent isopropanol and when using concentrated hydrochloric acid, butanol is used as the solvent and the process takes place in a slight vacuum at temperatures of 50 to 70°C under azeotropic distillation of the water content and setting of pH value of 1 to 3.

7. Process according to claim 1, characterised in that the 2,2-diphenyl-2-hydroxy-acetic acid-(2-dimethylamino-ethyl)-ester-hydrochloride is converted under heterogeneous conditions in aromatic, inert thinners, preferably toluol or xylol, with 1.7 equivalents of thionyl chloride at 90 to 95°C quantitatively into the α-chlorobenzilic-acid-ester-hydrochloride (III).

8. Process according to claim 1, characterised in that the 2,2-diphenyl-2-hydroxy-acetic acid-(2-dimethylamino-ethyl)-ester-hydrochloride is converted under homogenous conditions at 1.9 to 2.1 times the volume in weight of thionyl chloride based on the quantity of benzilic-acid-ester-hydrochloride (II) used at 5 to 90°C quantitatively into the α-chlorobenzilic-acid-ester-hydrochloride (III).

9. Process according to claim 1, characterised in that toluol is used as the co-solvent for etherisation of the α-chlorobenzilic acid-ester-hydrochloride (III).

10. Process according to claim 1, characterised in that the end product (IV) produced is re-crystallised from 1.6 to 2.2 times the quantity of methylethyl ketone using a pH value of 2 to 3.

## Revendications

1. Procédé de préparation technique du chlorhydrate de l'ester 2-diméthylamino-éthylique de l'acide 2,2-diphényl-2-(2-éthyl-butoxy) acétique (IV) par alkylation de l'acide benzilique ou de ses sels alcalins ou alcalino-terreux, chloration de l'ester d'acide benzilique ainsi formé et éthérification de l'ester de l'acide α-chlorobenzilique pour faire un produit final, procédé caractérisé en ce qu'on fait réagir l'acide benzilique ou son sel alcalin ou alcalino terreux avec le chlorhydrate de chlorure de 2-diméthylamino éthyle avec addition d'amines tertiaires, aliphatiques ou cycloaliphatiques, ayant une valeur de pKₛ supérieure à 9, ce qui permet d'obtenir, avec obtention du chlorhydrate de l'amine tertiaire, l'ester 2-diméthylamino éthylique de l'acide 2,2-diphényl-2-hydroxy-acétique (I), on transforme cet ester (I) en présence d'alcools primaires, secondaires ou tertiaires, à l'aide de chlorure d'hydrogène ou d'acide chlorhydrique concentré, en le chlorhydrate de l'ester 2-diméthylamino éthylique de l'acide 2,2-diphényl-2-hydroxy acétique (II), on transforme ce chlorhydrate (II), à l'aide de chlorure de thionyle, quantitativement en le chlorhydrate de l'ester 2-diméthylamino éthylique de l'acide 2,2-diphényl-2-chlor-acétique (III) et dans la dernière étape de réaction, on fait réagir ce chlorhydrate (III) avec le 2-éthyl-butanol en présence d'un solvant inerte non miscible à l'eau, bouillant au-dessous de 130°C, en opérant à des températures de réaction de 120 à 132°C pour obtenir le chlorhydrate de l'ester 2-diméthylamino-éthylique de l'acide 2,2-diphényl-2-(2-éthyl-butoxy) acétique.

2. Procédé selon la revendication 1, caractérisé en ce qu'on utilise 1,1 à 1,5 équivalent de chlorhydrate de chlorure de 2-diméthylamino éthyle, par rapport à la quantité d'acide benzilique ou de son sel que l'on met en oeuvre.

3. Procédé selon la revendication 1, caractérisé en ce qu'on fait réagir l'acide benzilique ou son sel avec le chlorhydrate de chlorure 2-diméthylamino éthyle avec addition d'amines tertiaires, aliphatiques ou cycloaliphatiques, ayant une valeur de pKₛ supérieure à 5 en opérant éventuellement en présence d'un solvant aprotique, apolaire, pour obtenir l'ester 2-diméthylamino éthylique de l'acide 2,2-diphényl-2-hydroxy-acétique.

4. Procédé selon la revendication 1, caractérisé en ce qu'on utilise comme amine tertiaire la triéthylamine, présente en un excès de 5 à 10 % par rapport à la quantité totale de chlorure à fixer.

5. Procédé selon la revendication 1, caractérisé en ce que, pour la première étape de réaction, on utilise du toluène ou du xylène comme solvant et l'on conduit la réaction dans un intervalle de température compris entre 50 et 150°C, en général sous reflux.

6. Procédé selon la revendication 1, caractérisé en ce que, lors de la transformation de l'ester d'acide benzilique (I) en son chlorhydrate à l'aide de chlorure d'hydrogène, qui est produit sur place ("in situ") ou est introduit ou ajouté directement, en opérant en présence du solvant isopropanol et en utilisant de l'acide chlorhydrique concentré comme solvant, on utilise du butanol et, ainsi, on travaille sous une légère dépression à des températures de 50 à 70°C en chassant par distillation un azéotrope de l'eau contenue et en ajustant le pH à une valeur de 1 à 3.

7. Procédé selon la revendication 1, caractérisé en ce que on transforme le chlorhydrate de l'ester 2-diméthylamino éthylique de l'acide 2,2-diphényl-2-hydroxy acétique, dans des conditions hétérogènes, dans des diluants aromatiques inertes, avantageusement le toluène ou le xylène, avec 1,7 équivalent de chlorure de thionyle à 90 à 95°C, quantitativement en le chlorhydrate de l'ester de l'acide α-chlorobenzilique (III).

8. Procédé selon la revendication 1, caractérisé en ce qu'on fait réagir le chlorhydrate de l'ester 2-diméthylamino éthylique de l'acide 2,2-diphényl-2-hydroxy acétique en opérant dans des conditions homogènes dans la quantité pondérale de 1,9 à 2,1 fois en chlorure de thionyle, par rapport à la quantité du chlorhydrate d'ester d'acide benzilique (II) mis en oeuvre, à 5 à 90°C, quantitativement pour obtenir le chlorhydrate de l'ester d'acide α-chlorobenzilique (III)

9. Procédé selon la revendication 1, caractérisé en ce que, pour l'éthérification du chlorhydrate d'ester de l'acide α-chlorobenzilique (III), on utilise du toluène comme co-solvant.

10. Procédé selon la revendication 1, caractérisé en ce que l'on soumet le produit final ainsi obtenu (IV) à une recristallisation dans 1,6 à 2,2 fois la quantité de méthyléthylcétone, en ajustant le pH à une valeur de 2 à 3.
